(19) Europäisches Patentamt
European Patent Office
Office européen des brevets

(11) Veröffentlichungsnummer : **0 159 508**
**B1**

(12) # EUROPÄISCHE PATENTSCHRIFT

(45) Veröffentlichungstag der Patentschrift : **27.05.87**

(51) Int. Cl.[4] : **C 07 C 21/09**, C 07 C 17/10

(21) Anmeldenummer : **85102683.1**

(22) Anmeldetag : **08.03.85**

(54) **Verfahren zur Herstellung von ungesättigten Dichlorisobutenen.**

(30) Priorität : **25.04.84 DE 3415335**

(43) Veröffentlichungstag der Anmeldung : **30.10.85 Patentblatt 85/44**

(45) Bekanntmachung des Hinweises auf die Patenterteilung : **27.05.87 Patentblatt 87/22**

(84) Benannte Vertragsstaaten : **AT BE CH DE FR GB IT LI NL**

(56) Entgegenhaltungen :
**JOURNAL OF THE AMERICAN CHEMICAL SOCIETY, Band 69, November 1947, Seiten 2614-2616, Easton, GB; J.F. HATCH et al.: "Allylic chlorides. III. Prepatation of the 1,3-dichloro-2-methyl-1-propenes"**

(73) Patentinhaber : **HÜLS AKTIENGESELLSCHAFT - RSP Patente / PB 15 - Postfach 13 20 D-4370 Marl 1 (DE)**

(72) Erfinder : **Müller, Dieter Jürgen, Dr. Stargarder Strasse 30 D-4370 Marl (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 159 508 B1

**Beschreibung**

Die Erfindung betrifft ein Verfahren zur Herstellung von ungesättigten Dichlorisobutenen, nämlich von cis- und trans-1,3-Dichlor-2-methyl-propen und 3-Chlor-2-chlormethyl-propen durch Umsetzung von 3-Chlor-2-methylpropen mit Sulfurylchlorid.

Von den genannten Produkten ist insbesondere das 3-Chlor-2-chlormethyl-propen als Zwischenprodukt für eine Reihe von organischen Synthesen interessant, während cis- und trans-Dichlor-2-methylpropen vorzugsweise zur Weiterchlorierung eingesetzt werden.

Die Herstellung der vorgenannten Dichlorisobutene ist nach der DD-PS 106 345 durch Gasphasenchlorierung von Isobuten bei 60 bis 80 °C möglich und liefert bei einem Isobuten/Chlor-Verhältnis von 0,4 bis 0,5 etwa 30 % 1,3-Dichlor-2-methyl-propen, 30 % 3-Chlor-2-chlormethylpropen, 30 % 1,2,3-Trichlor-2-methyl-propan und 10 % 3-Chlor-2-methyl-propen.

Andere Bildungsweisen bestehen darin, 3-Chlor-2-methyl-propen mittels Chlor bei niederen Temperaturen unter definierten Bedingungen weiter zu chlorieren, wobei etwa 25 bis 30 % 3-Chlor-2-chlormethylpropen, 25 % 1,3-Dichlor-2-methyl-propen, 30 % 1,2,3-Trichlor-2-methyl-propan und weitere höherchlorierte Produkte entstehen (Z. Chem. 8 (1968) 6, 220-221, J. Am. Chem. Soc. 69 (1947) 2 614-2 616 und Chem. Abstr. 63 (1965) 4 148).

Die Weiterchlorierung von 3-Chlor-2-methyl-propen mittels $SO_2Cl_2$ liefert nach dem Stand der Technik überwiegend das Additionsprodukt 1,2,3-Trichlor-2-methyl-propan, welches mit einer Ausbeute von 83 % anfällt (J. Am. Chem. Soc. 68 (1946) 787).

Diese Verfahren zur Herstellung von ungesättigten Dichlor-isobutenen führen somit zu unerwünschten Nebenprodukten. Dadurch ergab sich die Aufgabe, ein Verfahren zu finden, das die Herstellung von ungesättigten Dichlor-isobutenen in einfacher Weise mit höheren Ausbeuten ermöglicht.

Diese Aufgabe wird erfindungsgemäß entsprechend den Angaben der Patentansprüche gelöst.

Überraschenderweise ist nun gefunden worden, daß die Weiterchlorierung von 3-Chlor-2-methylpropen mit $SO_2Cl_2$ in Gegenwart von Aminen und/oder Phosphinen einen entgegengesetzten Reaktionsablauf zeigt und überwiegend Substitionsprodukte wie cis- und trans-1,3-Dichlor-2-methyl-propen und 3-Chlor-2-chlormethyl-propen mit Ausbeuten von insgesamt 70 bis 80 % liefert.

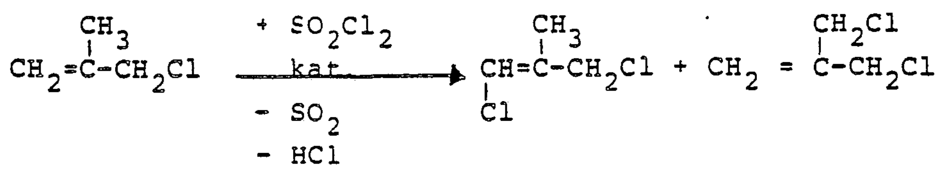


Die Umsetzung führt man bevorzugt in der Flüssigphase durch. Das Sulfurylchlorid kann man in stöchiometrischen Mengen einsetzen. Bevorzugt setzt man es im Unterschuß ein.

Bei der Ausgestaltung dieses überraschenden Reaktionsablaufes zum erfindungsgemäßen Verfahren hat es sich als zweckmäßig erwiesen, 3-Chlor-2-methyl-propen nach Zugabe der katalysierenden Stickstoff- und/oder Phosphor-Verbindung auf eine Starttemperatur von 30 bis 65 °C zu erwärmen und Sulfurylchlorid vorzugsweise im stöchiometrischen Unterschuß zuzudosieren. Äquivalente oder überstöchiometrische Mengen an $SO_2Cl_2$ stören den Reaktionsablauf an sich nicht. Allerdings ist dann die Aufarbeitung des Reaktionsgemisches erschwert, weil die Abtrennung von nicht umgesetztem $SO_2Cl_2$ schwieriger ist als die Abtrennung von nicht umgesetztem 3-Chlor-2-methyl-propen.

Da die Substitionsreaktion stark exotherm und unter dem Einfluß des Katalysators mit großer Geschwindigkeit abläuft, ist es zweckmäßig, die Zudosierung von $SO_2Cl_2$ in dem Maße vorzunehmen, wie es abreagiert. Der Reaktionsablauf kann entsprechend der Reaktionsgleichung über die Bildungsgeschwindigkeit des ausgasenden $SO_2$ und HCl verfolgt werden.

Die Temperatur der Reaktionsmischung kann bis an den Siedepunkt heranreichen. Die Abfuhr der Reaktionswärme kann sowohl über eine Mantelkühlung als auch über eine Verdampfungs-Rückflußkühlung erfolgen.

Die Reaktion kann sowohl diskontinuierlich, beispielsweise in einem Rührkessel als auch kontinuierlich, beispielsweise in einem Rohrreaktor oder einer Kaskade durchgeführt werden.

Als Katalysatoren haben sich organische Stickstoff- und Phosphorverbindungen als geeignet erwiesen, die als Amine bzw. Phosphine vorliegen oder sich davon ableiten.

Geeignete Katalysatoren sind zum Beispiel sowohl aliphatische Amine und Phosphine, wie beispielsweise Diisopropylamin, Triethylamin, Tirbutylamin, Tributylphosphin als auch aromatische Amine und Phosphine, wie beispielsweise Diphenylamin, oder heterocyclische Verbindungen wie Pyridin, Picoline, Pyrrol, Pyrazol, Chinolin, Chinaldin, Carbazol. Hierbei handelt es sich nur um eine beispielhafte Aufzählung.

Bevorzugt setzt man Pyridin, Picolin, Diisopropylamin, Triethylamin und/oder Chinolin ein.

Man kann auch Gemische der Amine und/oder Phosphine einsetzen.

In der Regel genügen etwa 1 bis 10 000 ppm dieser Verbindungen, um die Reaktion in der

gewünschten Weise zu katalysieren. Vorzugsweise werden 100 bis 1 000 ppm eingesetzt. Da vielfach Chlorkohlenwasserstoffe mit Aminen stabilisiert werden, kann ggf. auf diese Weise stabilisiertes 3-Chlor-2-methyl-propen bereits in der gewünschten Weise reagieren, ohne daß die zusätzliche Zugabe einer Stickstoffverbindung erforderlich wird.

Die Umsetzung erfolgt im allgemeinen bei einer Temperatur von 30 bis 70 °C, vorzugsweise bei 45 bis 65 °C in einer Zeit von 60 bis 180 min, vorzugsweise 90 bis 120 min. Die Zudosierzeit von $SO_2Cl_2$ beträgt im allgemeinen 10 bis 90 min, vorzugsweise 30 bis 60 min. Bevorzugt arbeitet man bei Normaldruck.

Zur Isolierung der ungesättigten Dichlorisobutene wird das Reaktionsprodukt einer Fraktionierung unterworfen, wobei es sich im Falle einer Normaldruckfraktionierung um die Fraktionen mit dem Siedebereich von 131 bis 133 °C (cis/trans-1,3-Dichlor-2-methyl-propen) und mit dem Siedebereich von 137 bis 139 °C (3-Chlor-2-chlormethyl-propen) handelt. Vorzugsweise wird die Fraktionierung allerdings unter vermindertem Druck durchgeführt, da die Produkte thermisch nicht völlig stabil sind.

Die Ausbeuten an den vorstehend genannten ungesättigten Dichlorisobutenen liegen im allgemeinen zwischen 70 und 80 %, bezogen auf umgesetztes 3-Chlor-2-methyl-propen.

Lichteinwirkung kann den Reaktionsablauf in Richtung Addition verschieben, so daß zweckmäßigerweise unter Lichtausschluß gearbeitet wird.

Das folgende Beispiel soll das erfindungsgemäße Verfahren verdeutlichen.

Beispiel 1

In einer Rührapparatur mit Rückflußkühler und Tropftrichter werden 90,6 g frisch destilliertes 3-Chlor-2-methyl-propen vorgelegt, mit einer Stickstoff- bzw. Phosphorverbindung entsprechend Tabelle 1 versetzt, auf 45 °C erwärmt und dann insgesamt 108 g Sulfurylchlorid unter Rühren in 30 bis 90 min zudosiert, wobei die Temperatur durch Thermostatisierung des Rührkolbens im Bereich zwischen 45 und 50 °C gehalten wird. Die bei der Reaktion gebildeten gasförmigen Nebenprodukte $SO_2$ und HCl werden über den Rückflußkühler abgezogen und in einer Vorlage mit auf pH 9,5 alkalisiertem Wasser absorbiert. Der pH-Wert wird durch automatische Titration mittels Natronlauge definierter Konzentrationen konstant gehalten, so daß der Reaktionsfortschritt anhand des Natronlaugeverbrauchs verfolgt werden kann. Die Sulfurylchloridumsätze liegen bei Reaktionszeiten von 90 min bis 180 min zwischen 90 und 98 %.

Die resultierende Produktmenge liegt zwischen 120 und 135 g. Das Rohprodukt wird nach Wasserwäsche und Trocknung über $K_2CO_3$ einer gaschromatografischen Analyse unterworfen. Die resultierende Produktzusammensetzung ist Tabelle 1 zu entnehmen, wobei das überschüssige, nicht umgesetzte 3-Chlor-2-methyl-propen rechnerisch eliminiert wird.

Die Beispiele sind hinsichtlich der Reaktionszeit, Reaktionstemperatur und Ausbeute nicht optimiert worden, da der prinzipielle Reaktionsablauf unter den erfindungsgemäßen Bedingungen aufgezeigt werden soll.

(Siehe Tabelle 1 Seite 4 f.)

Tabelle 1 : Beispiele zur katalytischen Umsetzung von 3-Chlor-2-methyl-propen mit $SO_2Cl_2$ in Gegenwart von organischen Stickstoff- und Phosphorverbindungen, vorliegend als Amine und Phosphine

| Amin bzw. Phosphin als Katalysator ppm | Reaktionstemperatur °C | Zudosierungszeit $SO_2Cl_2$ min | Reaktionszeit min | Rohproduktmenge g | Zusammensetzung des Reinproduktes % 1,2,3-Tri-chlor-2-methyl-propan | cis/trans-1,3-Dichlor-2-methyl-propen | 3-Chlor-2-chlor-methyl-propen |
|---|---|---|---|---|---|---|---|
| 1 Diisopropylamin 100 | 45 bis 50 | 30 | 90 | 125 | 27,5 | 35,5 | 36,5 |
| 2 Tributylamin 1 000 | 45 bis 50 | 30 | 90 | 124 | 23,0 | 37,0 | 39,0 |
| 3 Tributylphosphin 1 000 | 45 bis 50 | 60 | 150 | 124 | 23,0 | 37,5 | 38,5 |
| 4 Pyridin 1 000 | 45 bis 50 | 30 | 120 | 135 | 25,5 | 37,5 | 38,0 |
| 5 Chinaldin 1 000 | 45 bis 50 | 60 | 120 | 128 | 31,0 | 33,0 | 35,0 |
| 6 Carbazol 1 000 | 45 bis 50 | 90 | 180 | 125 | 22,0 | 38,0 | 40,0 |
| 7 Triphenylphosphin 1 000 | 45 bis 50 | 60 | 150 | 120 | 25,0 | 37,0 | 37,0 |

**Patentansprüche**

1. Verfahren zur Herstellung der ungesättigten Dichlor-isobutene 1,3-Dichlor-2-methyl-propen und 3-Chlor-2-chlormethyl-propen, dadurch gekennzeichnet, daß man 3-Chlor-2-methyl-propen mit Sulfurylchlorid in Gegenwart katalytisch wirkender Mengen an organischen Stickstoff- und/oder Phosphorverbindungen aus der Gruppe der Amine und/oder Phosphine umsetzt.

2. Verfahren nach Anspruch 1, dadurch gekennzeichnet, daß die Reaktion in der Flüssigphase durchgeführt wird.

3. Verfahren nach Anspruch 1 und 2, dadurch gekennzeichnet, daß die Reaktion bei 30 bis 70 °C, vorzugsweise bei 45 bis 65 °C, unter Normaldruck durchgeführt wird.

4. Verfahren nach Anspruch 1 bis 3, dadurch gekennzeichnet, daß Sulfurylchlorid, bezogen auf 3-Chlor-2-methyl-propen im stöchiometrischen Unterschuß eingesetzt wird.

5. Verfahren nach Anspruch 1 bis 4, dadurch gekennzeichnet, daß die organische Stickstoff- oder Phosphorverbindung aus der Gruppe der Amine oder Phosphine im Konzentrationsbereich von 1 bis 10 000 ppm zugesetzt wird oder ein bereits mit Aminen entsprechend stabilisiertes 3-Chlor-2-methyl-propen eingesetzt wird.

6. Verfahren nach Anspruch 1 bis 5, dadurch gekennzeichnet, daß man als Stickstoffverbindung aliphatische, aromatische oder heterocyclische Amine einsetzt.

7. Verfahren nach Anspruch 1 bis 5, dadurch gekennzeichnet, daß man als Phosphorverbindung aliphatische oder aromatische Phosphine einsetzt.

**Claims**

1. A process for the production of the unsatured dichloroisobutenes 1,3-dichloro-2-methylpropene and 3-chloro-2-chloromethylpropene, characterised in that 3-chloro-2-methylpropene is reacted with sulfuryl chloride in the presence of a catalytically effective amount of one or more organic nitrogen or phosphorus compounds selected from amines and phosphines.

2. A process according to claim 1, characterised in that the reaction is conducted in the liquid phase.

3. A process according to claim 1 or 2, characterised in that the reaction is carried out at 30 to 70 °C, preferably 45 to 65 °C, under standard pressure.

4. A process according to any of claims 1 to 3, characterised in that sulfuryl chloride is introduced in less than the stoichiometric amount relative to 3-chloro-2-methylpropene.

5. A process according to any of claims 1 to 4, characterised in that the organic nitrogen or phosphorus compound selected from amines and phosphines is added at a concentration in the range from 1 to 10,000 ppm or 3-chloro-2-methylpropene already correspondingly stabilised with amine is introduced.

6. A process according to any of claims 1 to 5, characterised in that an aliphatic, aromatic or heterocyclic amine is introduced as nitrogen compound.

7. A process according to any of claims 1 to 6, characterised in that an aliphatic or aromatic phosphine is introduced as phosphorus compound.

**Revendications**

1. Procédé de préparation des dichloro-isobutènes insaturés, en l'occurrence du 1,3-dichloro-2-méthyl-propène et du 3-chloro-2-chloro-méthyl-propène, caractérisé par le fait qu'en présence de quantité de composés organiques azotés et/ou phosphorés du groupe des amines et/ou des phosphines exerçant un effet catalytique, on fait réagir le 3-chloro-2-méthyl-propène sur du chlorure de sulfuryle.

2. Procédé selon la revendication 1, caractérisé par le fait qu'on effectue la réaction en phase liquide.

3. Procédé selon la revendication 1 et 2, caractérisé par le fait qu'on effectue la réaction sous la pression normale, à une température de 30 à 70 °C, de préférence de 45 à 65 °C.

4. Procédé selon les revendications 1 à 3, caractérisé par le fait qu'on utilise le chlorure de sulfuryle, relativement au 3-chloro-2-méthyl-propène, dans une quantité n'atteignant pas la quantité stoechiométrique.

5. Procédé selon les revendications 1 à 4, caractérisé par le fait qu'on ajoute le composé organique azoté ou phosphoré du groupe des amines ou des phosphines dans un domaine de concentration de 1 à 10 000 ppm, ou bien qu'on utilise un 3-chloro-2-méthyl-propène déjà stabilisé de façon correspondante avec des amines.

6. Procédé selon les revendications 1 à 5, caractérisé par le fait qu'on utilise, comme composé azoté, des amines aliphatiques, aromatiques ou hétéro-cycliques.

7. Procédé selon les revendications 1 à 5, caractérisé par le fait qu'on utilise, comme composé phosphoré, des phosphines aliphatiques ou aromatiques.